# EUROPÄISCHE PATENTSCHRIFT

(11) **EP 0 880 491 B1**
(45) Veröffentlichungstag und Bekanntmachung des Hinweises auf die Patenterteilung: **13.06.2001**
(21) Anmeldenummer: 97901073.3
(22) Anmeldetag: 21.01.1997
(51) Int. Cl.: C07C 29/80, C07C 29/94, C07C 31/20, C08G 63/12

(54) **VERFAHREN ZUR GEWINNUNG VON GLYKOLEN MIT NIEDRIGEM ALDEHYDGEHALT**
METHOD OF OBTAINING GLYCOLS HAVING A LOW ALDEHYDE CONTENT
PROCEDE D'OBTENTION DE GLYCOLS A FAIBLE TENEUR EN ALDEHYDE

(30) Priorität: 22.01.1996 DE 19602116
(43) Veröffentlichungstag der Anmeldung: 02.12.1998
(73) Patentinhaber: BASF AKTIENGESELLSCHAFT, 67056 Ludwigshafen (DE)
(72) Erfinder: MOHR, Jürgen, D-67269 Grünstadt (DE); VANSANT, Frans, B-2920 Kalmthout (BE)
(74) Vertreter: Kinzebach, Werner, Dr.
(86) Internationale Anmeldenummer: EP9700271
(87) Internationale Veröffentlichungsnummer: WO9727164

(56) Entgegenhaltungen:
- DE-B- 1 242 586
- CHEMICAL ABSTRACTS, vol. 81, no. 23, 9.Dezember 1974 Columbus, Ohio, US; abstract no. 151536c, Seite 476; XP002029878 & JP 49 051 212 A (MITSUI PETROCHEMICAL INDUSTRIES) 18.Mai 1974

## Beschreibung

Die vorliegende Erfindung betrifft ein Verfahren zur Gewinnung von Glykolen mit niedrigem Aldehydgehalt sowie Produkte, welche unter Verwendung dieser Glykole hergestellt werden.

Niedermolekulare Glykole, wie Mono-, Di- und Triethylenglykol, stellen wichtige Produkte der chemischen Industrie dar. Insbesondere Monoethylenglykol (ebenfalls bezeichnet als 1,2-Ethandiol, Ethylenglykol oder einfach MEG) zählt weltweit zu den Hauptprodukten der chemischen Industrie. Monoethylenglykol wird hauptsächlich als Frostschutzmittel für Kraftfahrzeugkühler sowie als Rohmaterial für die Herstellung von Polyestern verwendet.

Einziges derzeit für die technische Großproduktion von Ethylenglykol verwendetes Verfahren umfaßt die Hydrolyse von Ethylenoxid und die anschließende Aufarbeitung des dabei anfallenden Reaktionsgemisches. Die weltweite Produktionskapazität für Ethylenglykol über die Ethylenoxidhydrolyse wird derzeit auf 7 x 10⁶ Tonnen/Jahr geschätzt. Bei diesem Herstellungsverfahren wird das Ethylenoxid in speziellen Reaktoren und unter geeigneten Bedingungen kontinuierlich oder diskontinuierlich mit Wasser umgesetzt. Das so erhaltene wäßrige Reaktionsgemisch wird dann in mehreren Stufen aufkonzentriert und das Rohglykol schließlich durch Fraktionierung gereinigt (vgl. z.B.: K. Weissermel, H.-J. Arpe, Industrielle Organische Chemie, 3. Aufl., VCH 1988, S. 159 ff.). Das Reaktionsgemisch umfasst typischerweise als Hauptkomponenten Mono-, Di- und Triethylenglykol. Tetraethylenglykol und höhere Homologe sind üblicherweise in solch geringen Mengen vorhanden, daß sich eine Gewinnung in der Regel nicht lohnt.

Destillationsverfahren und Apparaturen für die Reinigung von Glykolen sind in unterschiedlichen Ausführungen bekannt (vgl. z.B.: Ullmanns Encyklopädie der technischen Chemie, 4. Auflage, VCH 1974, Band 8, S. 200 ff.). Meist werden hierbei mittels in Reihe geschalteter Destillations-Kolonnen zunächst Wasser, dann Ethylenglykol, und anschließend die höheren Glykolether kontinuierlich gewonnen. Als Verdampfer können bei den Destillationen unterschiedliche Aggregate verwendet werden; in moderneren kontinuierlichen Anlagen setzt man aber aus energetischen Gründen Fallfilmverdampfer ein. Aus Kostengründen sind diese Anlagenteile üblicherweise aus Kohlenstoff-Stahl hergestellt.

Die Destillationen finden wegen der hohen Siedepunkte von Ethylenglykol und seiner Homologen stets im Vakuum statt. Jede Vakuumdestillationsanlage - ob großtechnisch oder im Labormaßstab - besitzt eine gewisse Leckrate, d.h., Undichtigkeiten, die in geringem Maße den Zutritt der umgebenden Atmosphäre in die Anlage gestatten. Im laufenden Betrieb gerät so auch atmosphärischer Sauerstoff in die Vakuumdestillation.

Glykole lassen sich wie alle Alkohole sowohl thermisch (Autoxidation) als auch katalytisch leicht oxidieren. Die Umsetzungsprodukte von Ethylenglykol mit Sauerstoff oder anderen Oxidationsmitteln sind Aldehyde (Glykolaldehyd, Glyoxal, Formaldehyd, Acetaldehyd) sowie die korrespondierenden Säuren. Andererseits gelten jedoch insbesondere bei der Verwendung des Ethylenglykols zur Herstellung von Polyesterfolien besonders hohe Reinheitsanforderungen. Folien, die unter Verwendung von aldehydreichem Ethylenglykol hergestellt wurden, zeigen eine starke Lichtempfindlichkeit. Insbesondere ist eine leichte Vergilbung feststellbar. Die Anwesenheit dieser Oxidationsprodukte ist deshalb bei der Polyesterproduktion, aber auch in anderen Bereichen, ausgesprochen unerwünscht, so daß die Anwender bestrebt sind, mittels strenger Spezifikationen den Aldehydgehalt zu limitieren. Beispielsweise sollte der Aldehydgehalt in Ethylenglykol, das für die Folienherstellung bestimmt ist, weniger als 20 ppm betragen.

Solange bei der Ethylenglykol-Herstellung keine besonderen Effekte auftreten, ist die Menge der gebildeten Oxidationsprodukte normalerweise unbedeutend. Es wurde aber beobachtet, daß in technischen Anlagen ein Anstieg des Aldehydanteils im destillierten Glykol auftreten kann, der nicht ohne weiteres erklärbar war. Gleichzeitig fand man ungewöhnlich große Mengen an Rostpartikeln (Magnetit) in den Destillationssümpfen.

Das eben geschilderte Problem der verstärkten Aldehydbildung sollte auch bestehen bei der destillativen Reinigung von Ethylenglykol, das über andere Synthesewege, wie z.B. die katalytische Oxidation von Ethylen in Essigsäure, hergestellt wurde.

Außerdem fallen bekanntlich bei großtechnischen Prozessen, wie z.B. der Polyesterfaserherstellung, große Mengen Glykol-haltiger flüssiger Rückstände an. Eine Rückgewinnung der darin enthaltenen Glykole ist unter wirtschaftlichen Gesichtspunkten sinnvoll. Schließlich fallen große Mengen Ethylenglykol in Form von gebrauchter Kühlerflüssigkeit oder gebrauchter Frostschutzmittel an, woraus das Glykol ebenfalls wiedergewonnen werden kann. Bei der destillativen Aufarbeitung dieser Abfälle besteht aber ebenso das Problem einer unerwünschten Aldehydbildung. Hinzu kommt, daß die Abfälle bereits geringe Mengen Aldehyd enthalten können, wodurch die Herstellung reiner, d.h. im wesentlichen aldehydfreier Glykole zusätzlich erschwert wird.

Die JP-A-72/95106 beschreibt ein Verfahren zur Herstellung von Ethylenglycolen. Dabei erfolgt eine destillative Aufarbeitung in Gegenwart einer Organophosphit- bzw. Organophosphat-Verbindung, welche zur Verringerung des Aldehydgehalts in der Vorlage dient.

Die DE-AS-12 42 586 beschreibt die Stabilisierung von Lösungen mehrwertiger Alkohole durch Zugabe von Dinatriumhydrogenphosphit, mit dem Ziel einer Verringerung von Korrosionsschäden in den diese Alkohollösungen enthaltenden Behältern oder Vorrichtungen.

Es ist Aufgabe der vorliegenden Erfindung, ein Verfahren bereitzustellen, das die Gewinnung von Glykolen intechnischem Maßstab ermöglicht, deren Aldehydgehalt deutlich verringert ist. Insbesondere stellt sich die Aufgabe, Monoethylenglykol bereitzustellen, das die geltenden strengen Spezifikationen für Ausgangsprodukte zur Polyesterproduktion sicher erfüllt.

Der hierin verwendete. Ausdruck "Glykol" umfasst insbesondere Monoethylenglykol sowie die destillierbaren Homologen davon, wie Di-, Tri- und Tetraethylenglykol.

Überraschenderweise wurde die erfindungsgemäße Aufgabe gelöst durch Bereitstellung eines Verfahrens zur Gewinnung von Glykolen mit niedrigem Aldehydgehalt aus Glykol-haltigen Gemischen, wobei man die für die Glykol-Gewinnung verwendete Vorrichtung aus korrodierbarem Material ganz oder teilweise mit wenigstens einer reduzierenden Phosphorverbindung oberflächenbehandelt, indem man dem Glykol-haltigen Gemisch die Phosphorverbindung vor oder während der Aufarbeitung des Gemisches zusetzt; und anschließend das (die) Glykol(e) abdestilliert; und indem man gegebenenfalls die zur Aufarbeitung des Gemisches verwendete Vorrichtung ganz oder teilweise mit der reduzierenden Phosphorverbindung vorbehandelt. Es sollten zumindest diejenigen Anlagenteile, die im Verlauf der Aufarbeitung des Gemisches in Kontakt mit einem Glykol kommen, behandelt werden.

Die Oberflächenbehandlung soll insbesondere an den Innenflächen derjenigen Anlagenteile erfolgen, die während der Aufarbeitung des Gemischs mit einem dampfförmigen Glykol dauernd oder zeitweise in Kontakt stehen. Dies gilt insbesondere für Anlagenteile, in denen bei erhöhter Temperatur gearbeitet wird und welche aus korrodierbarem Material, wie z.B. Stahl, gefertigt sind. Dies kann beispielsweise der Fall sein bei Verdampfern und deren Zu- und Ableitungsrohren, Destillationskolonnen und Reaktoren. In all diesen Anlagenteilen können sich Dampfräume bilden, worin bei den dort herrschenden Temperaturen die unerwünschte Aldehydbildung ablaufen kann.

Erfindungsgemäß kann diese Oberflächenbehandlung diskontinuierlich oder kontinuierlich erfolgen. Bei diskontinuierlicher Arbeitsweise können die zur Auftrennung des Gemisches benötigten Anlagenteile vor der Verarbeitung des Gemisches ganz oder teilweise mit der Phosphorverbindung behandelt werden. So kann man z.B. die für die Vakuumdestillation von Monoethylenglykol verwendete Destillationskolonne mit der Phosphorverbindung behandeln, wenn die Herstellung von aldehydarmem Monoethylenglykol erfolgen soll. Für die diskontinuierliche Fahrweise kann die Oberflächenbehandlung beispielsweise vor Verarbeitung jeder aufzuarbeitenden Gemischcharge oder aber in anderen geeigneten Zeitintervallen erfolgen. Dies ist abhängig von der Stärke der bei der Trennung beobachteten Aldehyd-Neubildung.

Arbeitet man dagegen kontinuierlich, so kann dem glykolhaltigen Gemisch die Phosphorverbindung vor der Auftrennung zugesetzt werden. Das phosphorhaltige Gemisch kann dann auf der Anlage aufgetrennt werden. Besonders bevorzugt ist erfindungsgemäß die kontinuierliche Zudosierung der Phosphorverbindung zum Gemisch unmittelbar vor Auftrennung. Dies ist insbesondere dann empfehlenswert, wenn zu erwarten ist, daß das aufzutrennende Gemisch Aldehyd bereits vor der Auftrennung enthält. Diese Aldehyde können dadurch bereits vor dem eigentlichen Trennschritt durch die Phosphorverbindung reduziert werden. Will man z.B. aldehydarmes Monoethylenglykol herstellen, so kann man einem glykolhaltigen Gemisch, das man beispielsweise durch kontinuierliche Ethylenoxid-Hydrolyse erzeugt, die Phosphorverbindung unmittelbar vor Einleitung in die zur Monoethylenglykol-Abtrennung verwendete Vakuumdestillationskolonne kontinuierlich zusetzen.

Eine Kombination von kontinuierlicher und diskontinuierlicher Verfahrensweise ist ebenfalls denkbar.

Die erfindungsgemäß verwendete Phosphorverbindung kann eine anorganische oder organische Phosphorverbindung oder eine Kombination von anorganischen und organischen Verbindungen sein, die den gewünschten reduktiven Effekt zeigen. Die eingesetzte Phosphorverbindung umfaßt eine reduktive P⁺³-haltige Spezies. Vorzugsweise ist sie ausgewählt unter phosphoriger Säure (welche überwiegend in der stabilen tautomeren Form HP(O)(OH)₂ vorliegt, die auch als Phosphonsäure bezeichnet wird; vgl. Römpp Chemie Lexikon, 9. Aufl.) und den Salzen davon. Salze der Phosphorigen Säure sind vorzugsweise ausgewählt unter wasserlöslichen Salzen, wie insbesondere Alkalimetall-, Zink- und Calcium-Phosphiten. Besonders bevorzugte Alkalimetallphosphite sind Natrium- und Kaliumphosphit. Die entsprechenden Hydrogenphosphite sind ebenfalls verwendbar. Am meisten bevorzugt ist jedoch die Verwendung der Säure selbst.

Erfolgt die Zugabe der Phosphorverbindung zum Gemisch selbst, wird diese gewöhnlich so durchgeführt, daß die Phosphorverbindung in einem Anteil von etwa 10 bis etwa 5000 ppm vorliegt, bevor man Glykol aus dem Gemisch entfernt. Vorzugsweise sollte der Anteil im Bereich von etwa 100 bis etwa 1000 ppm, und insbesondere bei etwa 500 ppm liegen. Die Phosphorverbindung wird vorzugsweise gelöst in einem Glykol, wie Monoethylenglykol, zugesetzt.

Soll die Anlage diskontinuierlich mit der Phosphorverbindung oberflächenbehandelt werden, so stellt man eine Lösung her, welche die Phosphorverbindung in einem Anteil von etwa 0,1 bis 10 Gew.-%, vorzugsweise etwa 0,5 bis 5 Gew.-%, bezogen auf das Gesamtgewicht der Lösung, enthält. Die Phosphorverbindung kann in einem geeigneten organischen Lösungsmittel, wie z.B. Monoethylenglykol, oder in einem wäßrigen Lösungsmittel, wie z.B. Wasser, gelöst werden. Eine wäßrige Lösung wird bevorzugt verwendet. Mit der auf diese Weise hergestellten Lösung behandelt man dann diejenigen Anlagenteile, welche zur Auftrennung des Gemisches benötigt werden. Die Behandlung kann beispielsweise erfolgen, indem man die betreffenden Anlagenteile mit der Lösung flutet und über einen geeigneten Zeitraum inkubiert. Alternativ ist eine Spülung der Anlagenteile denkbar, wobei man die Lösung durch Umpumpen über einen geeigneten Zeitraum zirkulieren läßt. Außerdem ist ein Besprühen oder Berieseln der Anlagenteile denkbar. Die erforderliche Behandlungsdauer kann vom Fachmann ohne große Schwierigkeiten bestimmt werden. Sie kann z.B. im Bereich von etwa 2 bis 8 Std., wie etwa 4 bis 6 Std., liegen. Die Behandlung kann bei Umgebungstemperaturen oder z.B. mit erwärmter wässriger Lösung (z.B. auf 40 bis 80°C erwärmt) erfolgen.

Aufgrund der erfindungsgemäßen Aufarbeitung des glykolhaltigen Gemisches erhält man insbesondere Ethylenglykol mit überraschend geringem Aldehydgehalt, welcher im Bereich von weniger als etwa 20 ppm, wie z.B. bei etwa 10-20 ppm, liegt. Überraschenderweise gelingt es mit Hilfe des erfindungsgemäßen Verfahrens, die strengen Spezifikationen für Ethylenglykol, die insbesondere bei der Herstellung von Polyester-Fasern und -Folien gelten, sicher einzuhalten. Die erfindungsgemäß hergestellten Glykole finden daher insbesondere vorteilhaft Verwendung bei der Herstellung von Polyesterfasern und -folien.

Der erfindungsgemäß beobachtete überraschende Effekt kann, ohne darauf beschränkt zu sein, wie folgt erklärt werden:

Die Autoxidation von Ethylenglykol, also die direkte Umsetzung mit Sauerstoff ohne Katalysator, ist ein langsam ablaufender Prozeß. In Laborversuchen, bei denen Glykol unter den Bedingungen einer typischen Vakuumdestillation dem durch die natürliche Leckrate eindringenden Sauerstoff ausgesetzt ist, beobachtet man nur eine langsame und geringe Zunahme der Aldehydwerte. Dies gilt insbesondere dann, wenn diese Versuche in Apparaturen aus inerten Materialien (z.B. Glas) durchgeführt werden. Auch erzwungene Leckraten mit erhöhter Sauerstoffzufuhr führen zu keiner drastischen Verschlechterung dieser Werte.

Bringt man jedoch in die gleiche Versuchsanordnung Eisenkörper (z.B. Drehspäne) ein und zwar so, daß diese in Kontakt mit der Dampfphase über dem destillierten Glykol geraten können, dann beobachtet man bei gleicher Leckrate um ein Vielfaches erhöhte gewissem Umfang Korrosion auf, bei der in erster Linie Magnetit (Rost) gebildet wird.

Auch mit reinem Magnetit statt mit Eisenspänen beobachtet man die gleiche verstärkte Aldehydbildung. Befinden sich Eisenspäne oder Magnetit jedoch in Flüssigkeit (z.B. im Destillationssumpf), so daß kein Gasphasenkontakt möglich ist, dann bleibt auch die erhöhte Aldehydbildung aus.

Die Bildung von Magnetit ist als schützender Überzug in Anlagenteilen aus Eisen an sich bekannt und sogar erwünscht. Unter bestimmten Umständen können aber aus einem kompakten Magnetitüberzug Partikel herausgelöst werden, die, an anderer Stelle der Anlage angeschwemmt, katalytisch die Oxidation von Glykol begünstigen. Solche Magnetit-Partikel können in der Tat auch gelegentlich während laufender Prozesse in geringer Menge durch Filtration isoliert werden. Treten diese Partikel kontinuierlich auf, dann muß die Anlage auf anderem Wege vor Korrosion geschützt werden.

Durch die erfindungsgemäße Zugabe von phosphoriger Säure bzw. von Phosphiten in die Zuläufe der Destillationen können sowohl Korrosion unter Magnetitbildung als auch die unerwünschte Oxidation von Glykol verhindert werden.

Die vorliegende Erfindung wird nun anhand folgender Ausführungsbeispiele näher erläutert.

### Ausführungsbeispiele

Mit Hilfe der in den Beispielen 1 bis 4 beschriebenen Experimente wurde der Einfluß des Anlagen-Werkstoffs auf die Aldehydbildung aus Ethylenglykol untersucht:

### Beispiel 1

### Destillationsversuche mit Ethylenglykol

Zunächst wurde in einer einfachen Destillationsapparatur bestehend aus Siedeblase mit Siedekapillare, Füllkörperkolonne (1 = 40 cm; d = 2,5 cm), absteigendem Kühler, Vorlage und Einrichtung zur Vakuumerzeugung, Ethylenglykol ( 700-800 ml) bei 200 mbar und 150-160°C Sumpftemperatur destilliert. Es wurden 13 % Sumpf gelassen; die Versuchsdauer betrug ca. 2 h. Als Füllkörper für die Kolonne dienten Glas- oder Eisenringe. Über die Siedekapillare wurde wahlweise Luft oder Stickstoff eingeperlt.

Die Ergebnisse sind in folgender Tabelle 1 zusammengefaßt:

**Tabelle 1:**

| Destillation von Glykol über Glas oder Eisenfüllkörper | | | | | |
|---|---|---|---|---|---|
| Bedingung: | | Füllkörper | | | |
| | | Glas Eisen | | | |
| | | Aldehyd (ppm) ¹⁾ ³⁾ | | | |
| | | frei | gesamt²⁾ | frei | gesamt²⁾ |
| Luft | Destillat | 27 | 38 | 42 | 53 |
| | Sumpf | <5 | 51 | 30 | 55 |
| | | | | | |
| | Bilanz | 24 | 40 | 40 | 53 |
| Stick-stoff | Destillat | 14 | 25 | 24 | 30 |
| | Sumpf | <5 | 57 | 12 | 52 |
| | | | | | |
| | Bilanz | 13 | 29 | 22 | 33 |

| | | | | | |
|---|---|---|---|---|---|
| ¹⁾ Aldehyd-Ausgangswerte: frei = 15 ppm; gesamt = 22 ppm | | | | | |
| ²⁾ Der Unterschied zwischen freiem und Gesamtaldehyd ist der sogenannte "gebundene Aldehyd", der sich im vorliegenden Fall insbesondere in Form der Acetale der direkten Bestimmung entzieht. | | | | | |
| ³⁾ Die Bestimmung der Aldehydwerte erfolgte nach der "MBTH-Methode", einer photometrischen Methode für freien und gebundenen Aldehyd (analog zu E. Savicky et al, (1961) Analyt.Chem., 33; 93-96). | | | | | |

Wichtig für die Interpretation der Analysendaten sind vor allem die bilanzierten (d.h., unter Berücksichtigung des Sumpf: Destillat-Verhältnisses von 13:87 erhaltenen) Gesamtaldehydgehalte aus der Tabelle.

In Anwesenheit von Luft wird eine Erhöhung des Gesamtaldehydgehaltes beobachtet, die aber im Falle der Eisenfüllkörper deutlich stärker ausfällt. Außerdem bildet sich auf diesen Füllkörpern ein körniger, leicht beweglicher schwarzer Belag von Magnetit.

Wird die Destillation unter Stickstoff betrieben, ist die Aldehydbildung wesentlich geringer bis praktisch vernachlässigbar.

### Beispiel 2

### Rückflußversuche

In weiteren Versuchen wurde Glykol in der Apparatur gemäß Beispiel 1 und unter sonst weitgehend gleichen Bedingungen am Rückfluß, also ohne Abdestillation, erhitzt. Auf diese Weise kann über einen längeren Zeitraum der Kontakt zwischen Dampfraum und untersuchtem Werkstoff aufrecht erhalten werden, so daß die vermuteten Effekte in stärkerem Maße auftreten können.

Die Kolonne wurde entweder als reine Glaskolonne (leer) oder gefüllt mit Eisendrehspänen betrieben. Durch letztgenannte Versuchsanordnung wird ein "Rückflußkühler" aus Eisen bzw. ein entsprechender Verdampfer mit Gasphasenkontakt simuliert.

Die Ergebnisse sind in Tabelle 2 zusammengefaßt.

### Rückflußkochen von Glykol in Gegenwart von Eisenfüllkörpern in Gasphase

**Tabelle 2:**

| Rückflußkochen von Glykol in Gegenwart von Eisenfüllkörpern in Gasphase | | | |
|---|---|---|---|
| Versuch Nr. | Zeit (h) | Atmosphäre | Aldehyd¹⁾ ³⁾ [ppm] |
| 1 | 13 | Luft | 162 |
| 2 | 14 | Luft | 180 |
| 3 | 12 | Stickstoff | 40 |
| | | | |
| 4²⁾ | 20 | Luft | 38 |

| | | | |
|---|---|---|---|
| ¹⁾ Gesamtaldehyd | | | |
| ²⁾ Kontrollversuch in Glas | | | |
| ³⁾ Aldehyd-Anfangswert 23 ppm | | | |

Man beobachtet, daß Ethylenglykol unter Luftzutritt an der Eisenoberfläche in relativ kurzer Zeit sehr viel Aldehyd bildet.

Insbesondere ein Vergleich mit dem Blindversuch (Nr. 4), der trotz längerer Laufzeit (20 Stunden) lediglich einen Anstieg des Aldehydgehaltes um 15 ppm zeigt, verdeutlicht den Zusammenhang zwischen Aldehydbildung und Eisenoberfläche. Die Eisenoberfläche überzieht sich im Verlauf der Versuche in Gegenwart von Sauerstoff mit einer dunklen Oxidschicht.

### Beispiel 3

### Rückflußkochen in getauchter Phase

In einer Glasapparatur gemäß Beispiel 1 wurde reines Monoethylenglykol (MEG) am Rückfluß gekocht. (p = 200 mbar; T = 150 - 160°C). Im Sumpf wurden Eisenspäne vorgelegt, die vollständig getaucht waren, mit der Gasphase also nicht in Berührung kommen konnten. Wiederum erfolgte die Einperlung von Luft über die Siedekapillare. Tabelle 3 zeigt die gemessenen Gesamtaldehydwerte am Ende der Versuche.

**Tabelle 3:**

| Rückflußkochen von Glykol in Gegenwart von Eisenfüllkörpern in getauchter Phase | | | |
|---|---|---|---|
| Nr. | Material | Atmosphäre | Aldehyd¹⁾[ppm] |
| 1 | Glas | Luft | 38 |
| | | | |
| 2 | Glas + Eisen (getaucht) | Luft | 39 |

| | | | |
|---|---|---|---|
| ¹⁾ Anfangswerte 23 ppm | | | |

Demnach verhält sich Eisen in getauchter Phase in gleicher Weise wie die leere Glasapparatur; die verstärkte Aldehydbildung in den vorangegangenen Versuchen muß daher in der Gasphase ablaufen.

### Beispiel 4

### Vorbehandlung mit Phosphoriger Säure

In zwei parallelen Versuchen wurde eine Glaskolonne mit folgenden Füllkörpern befüllt:
(1) unbehandelte Drehspäne
(2) Drehspäne, die vorher in einer 1-%igen Lösung von Phosphoriger Säure in Glykol über Nacht gelagert worden waren.

Die Versuchsdurchführung erfolgte gemäß Beispiel 2.

Die Ergebnisse sind in Tabelle 4 zusammengefaßt.

**Tabelle 4:**

| Wirkung einer Vorbehandlung der Füllkörper mit Phosphoriger Säure | | | |
|---|---|---|---|
| Nr. | Behandlung | Dauer [h] | Gesamtaldehyd [ppm]¹⁾ |
| 1 | / | 13 | 162 |
| | | | |
| 2 | H₃PO₃ | 45 | 40 |

| | | | |
|---|---|---|---|
| ¹⁾ Anfangswerte 23 ppm | | | |

Unter Luftzutritt entsteht im Falle der unbehandelten Eisenspäne Aldehyd bei gleichzeitiger Bildung und Ablagerung von Magnetit.

Im Falle der behandelten Eisenspäne tritt diese Korrosion praktisch nicht auf und die flüssige Phase bleibt fast klar.

Der Anteil an neugebildetem Aldehyd liegt deutlich niedriger als im ersten Fall.

Bei der Behandlung mit Phosphoriger Säure überziehen sich die Drehspäne mit einem grünen Belag, der über die Versuchsdauer erhalten bleibt.

### Beispiel 5

### Betriebsversuch

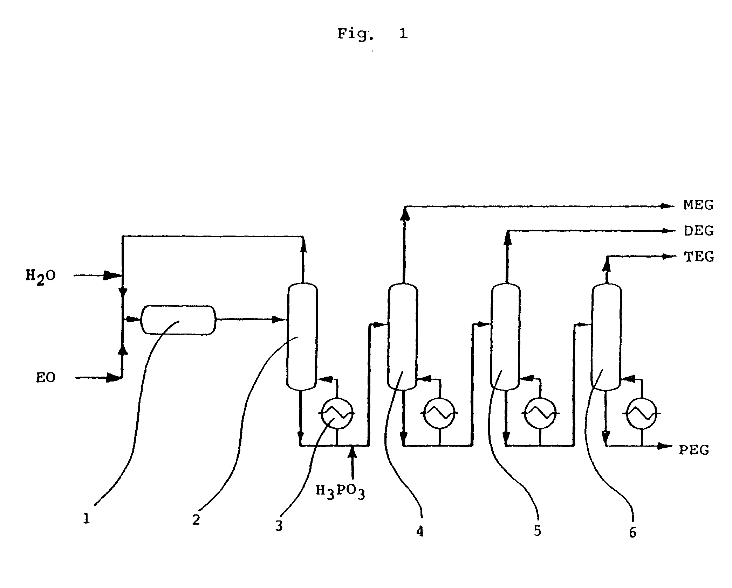

Ein Betriebsversuch wurde in einer kontinuierlich arbeitenden Produktionsanlage durchgeführt, die im wesentlichen nach dem in beiliegender Fig. 1 gezeigten Schema arbeitet. Nach Umsetzung der Reaktanden H₂O und Ethylenoxid (EO) im Reaktor (1) und Entwässerung des Rohproduktes in der Trocknerkolonne (2) wird dem Ethylenglykolgemisch nach Passieren des Verdampfers (3) und vor Eintritt in die Destillationskolonne (4) Phosphorige Säure (gelöst in Monoethylenglykol) in einer Konzentration von 20 ppm kontinuierlich zudosiert. Man beobachtet ein deutliches Absinken des Gesamtaldehydgehalts im reinen Monoethylenglykol (MEG) (vgl. Tabelle 5).

**Tabelle 5:**

| Verringerung des Gesamtaldehydgehalts in großtechnisch hergestelltem Monoethylenglykol | | |
|---|---|---|
| Aldehyd im Reaktionsgemisch | Aldehyd in MEG-Fraktion | Gleichgewichtseinstellung nach |
| 10 - 30 ppm | < 10 ppm | 2 Stunden |

Gleichzeitig vermindert sich auch die Menge des aus dem Sumpfablauf der Destillation abfiltrierbaren Rosts bzw. Magnetits.

Gewünschtenfalls kann H₃PO₃ z.B. auch unmittelbar vor den Destillationskolonnen (5) und(6) zudosiert werden, falls dies die Herstellung von Diethylenglykol (DEG) oder Triethylenglykol (TEG) mit niedrigem Aldehydgehalt erfordert.

## Patentansprüche

1. Verfahren zur Gewinnung von Glykolen mit niedrigem Aldehydgehalt aus Glykol-haltigen Gemischen, dadurch gekennzeichnet, daß man eine für die Glykol-Gewinnung verwendete Vorrichtung aus korrodierbarem Material ganz oder teilweise mit wenigstens einer reduzierenden Phosphorverbindung oberflächenbehandelt, indem man dem Glykol-haltigen Gemisch die Phosphorverbindung vor oder während der Aufarbeitung des Gemisches zusetzt; und anschließend das (die) Glykol(e) abdestilliert; und indem man gegebenenfalls die zur Aufarbeitung des Gemisches verwendete Vorrichtung ganz oder teilweise mit der reduzierenden Phosphorverbindung vorbehandelt.

2. Verfahren nach Anspruch 1, dadurch gekennzeichnet, daß die Vorbehandlung diskontinuierlich wenigstens in solchen Bereichen der Vorrichtung erfolgt, die während der Aufarbeitung mit dampfförmigem Glykol dauernd oder zeitweise in Kontakt stehen.

3. Verfahren nach Anspruch 1, dadurch gekennzeichnet, daß man dem Glykol-haltigen Gemisch die Phosphorverbindung in einem Anteil von etwa 10 bis etwa 5000 ppm kontinuierlich zudosiert, gegebenenfalls mischt und anschließend das (die) Glykol(e) aus dem Gemisch abtrennt.

4. Verfahren nach einem der Ansprüche 1 oder 2, dadurch gekennzeichnet, daß man die Vorrichtung ganz oder teilweise mit einer Lösung diskontinuierlich vorbehandelt, welche die Phosphorverbindung in einem Anteil von etwa 0,1 bis etwa 10 Gew.-% enthält.

5. Verfahren nach Anspruch 4, dadurch gekennzeichnet, daß man die Vorrichtung ganz oder teilweise mit der Lösung spült, flutet, berieselt oder besprüht.

6. Verfahren nach einem der vorhergehenden Ansprüche, dadurch gekennzeichnet, daß die Phosphorverbindung ausgewählt ist unter Phosphoriger Säure und den Salzen davon.

7. Verfahren nach Anspruch 6, dadurch gekennzeichnet, daß das Salz der Phosphorigen Säure ausgewählt ist unter wasserlöslichen Salzen, insbesondere Alkalimetall-, Calcium- und Zink-Phosphiten.

8. Verfahren nach einem der vorhergehenden Ansprüche, dadurch gekennzeichnet, daß das zu verarbeitende Glykol-haltige Gemisch ein Reaktionsgemisch aus der Ethylenglykolherstellung ist.

9. Verfahren nach einem der Ansprüche 1 bis 7, dadurch gekennzeichnet, daß das zu verarbeitende Glykol-haltige Gemisch ausgewählt ist unter Flüssigabfällen, wie gebrauchten Kühler- und Frostschutzflüssigkeiten, sowie Rückständen aus der chemischen Produktion.

10. Verfahren nach einem der vorhergehenden Ansprüche, dadurch gekennzeichnet, daß man Monoethylenglykol, Diethylenglykol und/oder Triethylenglykol mit jeweils niedrigem Aldehydgehalt erhält.

## Claims

1. A process for obtaining glycols with low aldehyde content from glycol-containing mixtures, which comprises surface-treating some or all of an apparatus made of corrodable material and used for glycol recovery with at least one reductive phosphorus compound by adding the phosphorus compound to the glycol-containing mixture before or during the working up of the mixture; and subsequently removing the glycol(s) by distillation; and, if desired, by pretreating some or all of the apparatus used for working up the mixture with the reductive phosphorus compound.

2. A process as claimed in claim 1, wherein pretreatment takes place discontinuously at least in those areas of the apparatus which, permanently or temporarily, are in contact with glycol in vapor form in the course of working up.

3. A process as claimed in claim 1, wherein the phosphorus compound is continuously metered into the glycol-containing mixture in a proportion of from about 10 to about 5000 ppm, mixing is carried out if desired, and then the glycol(s) is(are) separated off from the mixture.

4. A process as claimed in either of claims 1 and 2, wherein some or all of the apparatus is discontinuously pretreated with a solution which contains the phosphorus compound in a proportion of from about 0.1 to about 10% by weight.

5. A process as claimed in claim 4, wherein the apparatus, in whole or in part, is flushed, flooded, irrigated or sprayed with the solution.

6. A process as claimed in any of the preceding claims, wherein the phosphorus compuond is selected from phosphorous acid and the salts thereof.

7. A process as claimed in claim 6, wherein the salt of phosphorous acid is selected from water-soluble salts, especially alkali metal phosphites, calcium phosphites and zinc phosphites.

8. A process as claimed in any of the preceding claims, wherein the glycol-containing mixture to be processed is a reaction mixture from the production of ethylene glycol.

9. A process as claimed in any of claims 1 to 7, wherein the glycol-containing mixture to be processed is selected from liquid wastes, such as used radiator fluid and antifreeze liquids, and from residues from chemical production.

10. A process as claimed in any of the preceding claims, wherein monoethylene glycol, diethylene glycol and/or triethylene glycol each of low aldehyde content are obtained.

## Revendications

1. Procédé d'extraction de glycols ayant une faible teneur en aldéhydes, à partir de mélanges contenant du glycol, caractérisé en ce que l'on traite en surface, totalement ou partiellement, un dispositif utilisé pour l'extraction de glycol, constitué de matière attaquable par corrosion, avec au moins un composé phosphoré réducteur, en ajoutant au mélange contenant du glycol le composé phosphoré avant ou pendant le traitement du mélange; et en retirant ensuite par distillation le ou les glycols; et en prétraitant éventuellement, totalement ou partiellement, le dispositif utilisé pour le traitement du mélange, avec le composé phosphoré réducteur.

2. Procédé selon la revendication 1 caractérisé en ce que le pré-traitement a lieu en discontinu au moins dans. les zones du dispositif qui sont en contact permanent ou temporaire avec le glycol sous forme de vapeur pendant le traitement.

3. Procédé selon la revendication 1 caractérisé en ce que l'on ajoute en dosant en continu, le composé phosphoré à raison d'environ 10 à environ 5 000 ppm dans le mélange contenant le glycol, on le mélange éventuellement, puis on sépare le ou les glycols du mélange.

4. Procédé selon l'une quelconque des revendications 1 ou 2, caractérisé en ce que le dispositif est prétraité en discontinu, totalement ou partiellement, avec une solution qui contient le composé phosphoré à raison d'environ 0,1 à environ 10% en poids.

5. Procédé selon la revendication 4, caractérisé en ce que l'on rince, l'on inonde, totalement ou partiellement, le dispositif avec la solution, ou bien en la laissant ruisseler ou en la pulvérisant.

6. Procédé selon l'une quelconque des revendications précédentes caractérisé en ce que le composé phosphoré est choisi parmi l'acide phosphoreux et ses sels.

7. Procédé selon la revendication 6, caractérisé en ce que le sel de l'acide phosphoreux est choisi parmi les sels hydrosolubles, en particulier les phosphites de métal alcalin, du calcium et du zinc.

8. Procédé selon l'une quelconque des revendications précédentes caractérisé en ce que le mélange à traiter contenant du glycol, est un mélange réactionnel provenant de la préparation de l'éthylèneglycol.

9. Procédé selon l'une quelconque des revendications 1 à 7 caractérisé en ce que le mélange à traiter contenant du glycol est choisi parmi les rejets liquides, tels que les liquides usés de radiateur et d'antigel ainsi que les rejets de la production chimique.

10. Procédé selon l'une quelconque des revendications précédentes caractérisé en ce que l'on obtient le monoéthylèneglycol, le diéthylèneglycol et/ou le triéthylèneglycol avec, à chaque fois, une faible teneur en aldéhydes.
